# FASCICULE DE BREVET EUROPEEN

(11) **EP 1 755 494 B1**
(45) Date de publication et mention de la délivrance du brevet: **02.08.2017**
(21) Numéro de dépôt: 05762649.1
(22) Date de dépôt: 04.05.2005
(51) Int. Cl.: A61F 2/16

(54) **DISPOSITIF DE CONDITIONNEMENT POUR INJECTEUR DE LENTILLE INTRAOCULAIRE**
KONDITIONIERUNGSVORRICHTUNG FÜR EINEN INTRAOCULARLINSENINJEKTOR
CONDITIONING DEVICE FOR INJECTING THE INTRAOCULAR LENS

(30) Priorité: 06.05.2004 FR 0450874
(43) Date de publication de la demande: 28.02.2007
(73) Titulaire: Laboratoire de Contactologie Appliquée - LCA, 28000 Chartres (FR)
(72) Inventeur: VINCENT - AUBRY, Françoise, 28130 MEVOISINS (FR)
(74) Mandataire: CAPRI
(86) Numéro de dépôt international: PCT/FR2005/050303
(87) Numéro de publication internationale: WO 2005/110289

(56) Documents cités:
- WO-A-03/044946
- WO-A-03/049645
- WO-A-2004/052241
- FR-A- 2 789 890
- US-A- 4 787 904

## Description

La présente invention concerne un dispositif de conditionnement pour injecteur de lentille intraoculaire, et plus particulièrement un ensemble comprenant un tel dispositif de conditionnement et un tel injecteur.

L'utilisation d'injecteur pour réaliser l'injection d'une lentille intraoculaire, par exemple pendant une opération ophtalmologique de la cataracte, est bien connue. Pour améliorer ces systèmes, il a été proposé de réaliser des injecteurs à usage unique préemballés, de sorte que l'utilisateur n'a plus qu'à ouvrir l'emballage qui contient l'injecteur pour pouvoir directement l'utiliser et injecter la lentille. La lentille peut être placée dans une cassette à insérer dans l'injecteur ou directement être placée dans le corps dudit injecteur. Des moyens de distribution, comportant généralement un piston déplaçable manuellement, sont prévus pour permettre à l'utilisateur de distribuer ladite lentille à travers ledit injecteur directement dans l'oeil d'un patient lors d'une opération chirurgicale ophtalmologique. Ce genre de dispositif doit bien entendu être stocké de manière stérile avant son utilisation. Il a été proposé de remplir de liquide l'intérieur de l'injecteur, et de munir l'embout dudit injecteur d'un bouchon. Il s'est avéré que ce type de dispositif a tendance à se vider à long terme, notamment en raison d'une part de l'insuffisance d'étanchéité des joints et d'autre part de la barrière de vapeur du polypropylène, matériau généralement utilisé pour réaliser les injecteurs, qui est loin d'être parfaite. Il a également été proposé de disposer les injecteurs dans une poche souple remplie d'une solution ou liquide de stockage. Un tel ensemble est par exemple divulgué dans le document US 4 787 904. Toutefois, cette mise en oeuvre présente des inconvénients, concernant notamment des difficultés pour garantir une protection mécanique correcte de l'injecteur qui est fragile, ou pour réaliser de manière appropriée la stérilisation. En effet, cette stérilisation est généralement réalisée par chaleur humide dans un autoclave à pression de vapeur d'eau, et les écarts de pression et de température qui se produisent en fin de stérilisation présentent des risques pour l'intégrité desdites poches souples, ce qui nécessite l'utilisation d'appareils de stérilisation plus complexes fonctionnant avec pression et contre-pression ou avec douche d'eau surchauffée. Par ailleurs, la réalisation d'un double emballage stérile tel que requis pour la chirurgie n'est pas guère envisageable avec les poches souples. De même, l'utilisation de poches souples peut présenter un risque d'évaporation à travers la paroi des récipients, ce qui peut être préjudiciable au maintien à long terme des propriétés de l'injecteur. En effet, une perte de liquide, par exemple par évaporation à travers ladite poche souple du liquide de stockage, peut entraîner une diminution des propriétés de l'injecteur lui-même, si celui-ci entre en contact avec de l'air par exemple. Entre autre, on peut citer des problèmes de vieillissement de la matière plastique ainsi que le risque d'une perte de liquide contenu à l'intérieur de l'injecteur lui-même. Les conditionnements réalisés par des poches souples sont de plus sensibles aux effractions accidentelles, par exemple pendant le transport ou lors d'autres manipulations, et il n'est pas toujours évident pour l'utilisateur final de vérifier l'intégrité absolue du conteneur de l'injecteur au moment de son utilisation. Les microfuites ne sont par exemple pas toujours visibles lorsque des poches souples sont utilisées. De même, un stockage de l'injecteur dans une poche souple remplie de liquide n'est pas très pratique au moment de l'ouverture par l'utilisateur final, les risques de fuite dudit liquide au moment de l'ouverture étant importants. Le document WO2004052241 représente l'état de la technique selon l'article 54(3) CBE et divulgue un ensemble comportant un injecteur de lentille intraoculaire et un dispositif de conditionnement rempli d'une solution pour recevoir et stocker ledit injecteur en immersion totale. La présente invention a pour but de fournir un ensemble comportant un injecteur de lentille intraoculaire et un dispositif de conditionnement qui ne reproduit pas les inconvénients susmentionnés.

Plus particulièrement, la présente invention a pour but de fournir un tel ensemble qui soit parfaitement étanche à l'eau et à la vapeur d'eau, même pendant une longue durée de stockage.

La présente invention a aussi pour but de fournir un tel ensemble qui résiste au choc et qui évite toute détérioration de l'injecteur contenu dans le dispositif de conditionnement, pendant le transport ou tout autre manipulation réalisée avant l'utilisation finale de l'injecteur.

La présente invention a encore pour but de fournir un tel ensemble qui soit simple et peu coûteux de fabrication et d'assemblage, et facile d'utilisation pour l'utilisateur final.

La présente invention a également pour but de fournir un tel ensemble qui soit facile et sure à stériliser de manière optimale.

La présente invention a donc pour objet l'ensemble selon la revendication 1, ledit ensemble comportant un injecteur de lentille intraoculaire comportant un corps qui contient une lentille intraoculaire, et un dispositif de conditionnement, ledit dispositif de conditionnement étant destiné à recevoir et stocker ledit injecteur avant son utilisation, ledit dispositif de conditionnement étant réalisé dans un matériau étanche à l'eau, et étant rempli d'une solution de stockage pour maintenir au moins le corps dudit injecteur et la lentille intraoculaire en immersion totale pendant toute la durée du stockage, ledit dispositif de conditionnement comportant un réservoir sensiblement rigide et une ouverture pour mettre en place et retirer ledit injecteur, ledit réservoir comportant au moins un épaulement interne coopérant avec une partie de l'injecteur pour le centrer et maintenir son extrémité d'éjection de la lentille en éloignement des parois dudit réservoir, ledit dispositif de conditionnement comportant un réservoir du type blister fermé par une membrane étanche notamment en aluminium.

Avantageusement, ladite solution de stockage est de l'eau, une solution saline physiologique, ou une solution viscoélastique.

Avantageusement, ledit matériau est du verre.

En variante, ledit matériau est un matériau synthétique à haute étanchéité à la vapeur.

Avantageusement, le remplissage du dispositif de conditionnement avec la solution de stockage est réalisé de telle sorte que la totalité du corps de l'injecteur est immergée dans toutes les positions dudit dispositif de conditionnement.

Avantageusement, ledit injecteur comporte un corps comprenant une partie environ cylindrique recevant la lentille de manière sensiblement non-déformée, une partie intermédiaire environ tronconique pour plier la lentille, et une partie d'extrémité d'éjection de la lentille, l'injecteur comprenant également des moyens de distribution déplaçables manuellement dans ledit corps pour déplacer la lentille de la partie environ cylindrique du corps vers la partie d'extrémité d'éjection du corps, et pour ensuite l'éjecter, ledit corps comportant à son extrémité opposée à l'extrémité d'éjection une bride radiale adaptée à coopérer avec un épaulement interne prévu dans le dispositif de conditionnement pour positionner ledit injecteur dans ledit dispositif de conditionnement de telle sorte que l'extrémité d'éjection est éloignée des parois du dispositif de conditionnement et que le corps est totalement immergé dans ladite solution de stockage, en toutes positions dudit dispositif de conditionnement.

Avantageusement, ledit dispositif de conditionnement est sensiblement transparent.

Avantageusement, ledit ensemble est adapté à être soumis à une stérilisation par chaleur humide, notamment dans un autoclave à pression de vapeur d'eau.

D'autres caractéristiques et avantages de l'invention apparaîtront plus clairement au cours de la description détaillée suivante, faite en référence aux dessins joints donnés à titre d'exemples non limitatifs et sur lesquels :
- la figure 1 est une vue schématique en section transversale d'un dispositif de conditionnement pour injecteur intraoculaire selon un premier mode de réalisation de présente invention ;
- la figure 2 est une vue schématique similaire à celle de la figure 2 montrant une variante de réalisation du premier mode de réalisation de la figure 1 ;
- la figure 3 est une vue schématique similaire à celles des figures 1 et 2, d'un second mode de réalisation de la présente invention ;
- la figure 4 est une vue schématique en section transversale d'une variante de réalisation du second mode de réalisation représenté sur la figure 3 ; et
- la figure 5 est une vue similaire à celle de la figure 4, prise selon un autre angle de vue.

Selon l'invention, l'ensemble comporte un injecteur 10 contenant une lentille intraoculaire et un dispositif de conditionnement 20, comportant un réservoir 21, 22.

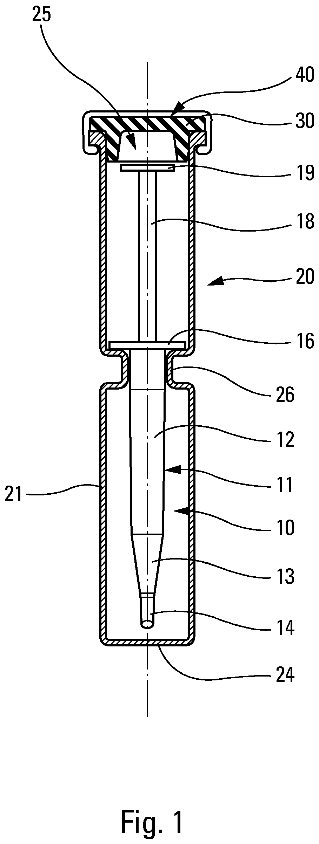

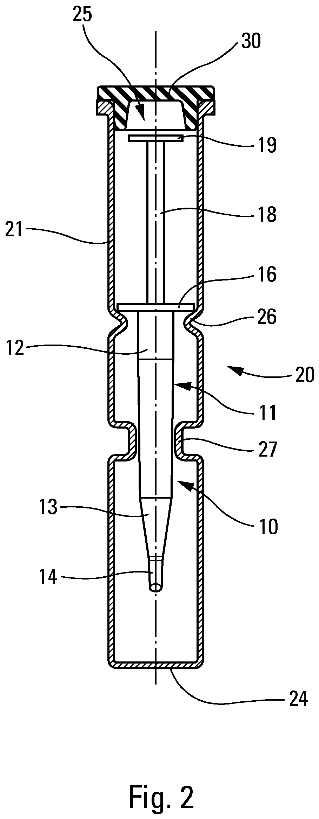

Les figures 1 et 2 montrent deux variantes d'un premier mode de réalisation. Dans ce premier mode de réalisation, le dispositif de conditionnement 20 comporte un réservoir 21, de préférence rigide, avantageusement réalisé sous la forme d'un flacon comportant un bouchon 30. Ce bouchon 30 est placé dans une ouverture 25 dudit flacon 21 et il peut avantageusement être fixé sur ledit flacon par une capsule sertie 40, comme représenté en figure 1. Le bouchon 30 peut être un bouchon élastomère tel qu'agréé pour les perfusions ou similaire.

Avantageusement, le flacon 21 est réalisé en verre, qui est un matériau parfaitement étanche à l'eau et à la vapeur, et dans lequel il n'y a pas de perte de liquide, même après un très long temps de stockage, typiquement de plusieurs années. En variante, on pourrait utiliser aussi des matériaux synthétiques étanches, par exemple des matériaux plastiques haute performance qui donnent à la fois une bonne rigidité au flacon et une parfaite barrière à l'évaporation du liquide. Le réservoir ou flacon 21 est rempli d'une solution de stockage qui peut être de l'eau, une solution saline physiologique ou une solution viscoélastique telle qu'utilisée en chirurgie ophtalmique. Avantageusement, le réservoir 21 comporte au moins un épaulement interne coopérant avec une partie de l'injecteur 10, à la fois pour le centrer et pour maintenir son extrémité d'éjection de la lentille en éloignement des parois du réservoir 21. Plus précisément, l'injecteur tel que représenté sur les dessins, comporte avantageusement un corps 11 pourvu d'une partie cylindrique 12, une partie d'éjection de lentille 14 formant l'embout à travers lequel la lentille est éjectée, et une partie tronconique 13 qui relie ladite partie cylindrique 12 et à ladite partie d'éjection 14. Une bride radiale 16 est avantageusement prévue à l'extrémité de ladite partie cylindrique 12 du corps 11 qui est opposée à l'embout d'éjection 14. Des moyens de distribution, comportant notamment un piston 18 pourvu d'une surface d'actionnement plane 19 sont avantageusement prévus pour réaliser l'éjection de la lentille (non représentée) disposée dans le corps de l'injecteur. De manière générale, la structure de l'injecteur, tel que décrite ci-dessus, ressemble à celle d'une seringue.

Dans ce mode de réalisation, un épaulement interne 26 est avantageusement prévu dans le flacon ou réservoir 21 pour coopérer avec ladite bride radiale 16 de l'injecteur. Cette mise en oeuvre garantit, comme on le voit sur les figures 1 et 2, d'éviter tout contact entre l'embout d'éjection 14, qui est une partie sensible de l'injecteur, avec les parois du réservoir 21, et notamment sa paroi inférieure 24. Un tel positionnement de l'injecteur garantit ainsi une protection efficace de l'embout d'éjection qui ne pourra pas s'entrechoquer avec les parois du flacon, et en particulier ne pourra pas heurter la paroi inférieure de ce flacon. Ainsi, cet épaulement interne favorise la création d'un espace confiné sécurisé pour l'injecteur 10 et évite donc toute possibilité d'altérations de l'embout d'éjection, notamment lors d'un transport de l'ensemble injecteur-dispositif de conditionnement. Avantageusement, un second épaulement interne 27 peut être prévu pour favoriser encore davantage le positionnement de l'injecteur à l'intérieur dudit flacon 21, comme cela apparaît notamment sur la figure 2. Bien entendu, les figures 1 et 2 ne montrent que des exemples de réalisation particuliers, et d'autres mises en oeuvres sont donc possibles.

Avantageusement, la solution de stockage est remplie à l'intérieur du flacon 21 jusqu'à un niveau tel que même lorsque le flacon 21 est dans une position autre que la position droite représentée sur les figures 1 et 2, la totalité du corps 11 et la lentille sont en immersion totale à l'intérieur du réservoir. Dans le mode de réalisation des figures 1 et 2, ceci signifie que le liquide doit être rempli dans le réservoir 21 de tel sorte que même lorsque le réservoir 21 est en position inversée, avec l'embout d'éjection 14 situé en haut, le niveau de liquide se situera toujours au-dessus dudit embout d'éjection 14. Avantageusement, le réservoir ou flacon 21 est transparent, ce qui permet à l'injecteur d'être clairement visible de l'extérieur.

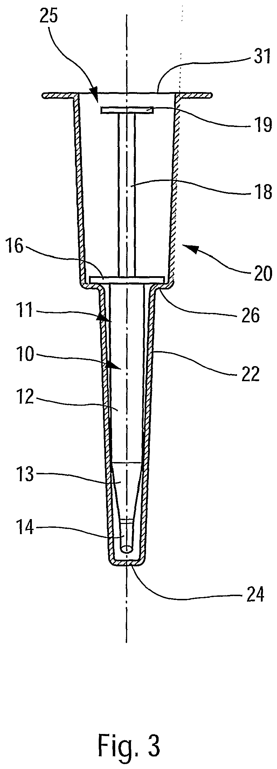

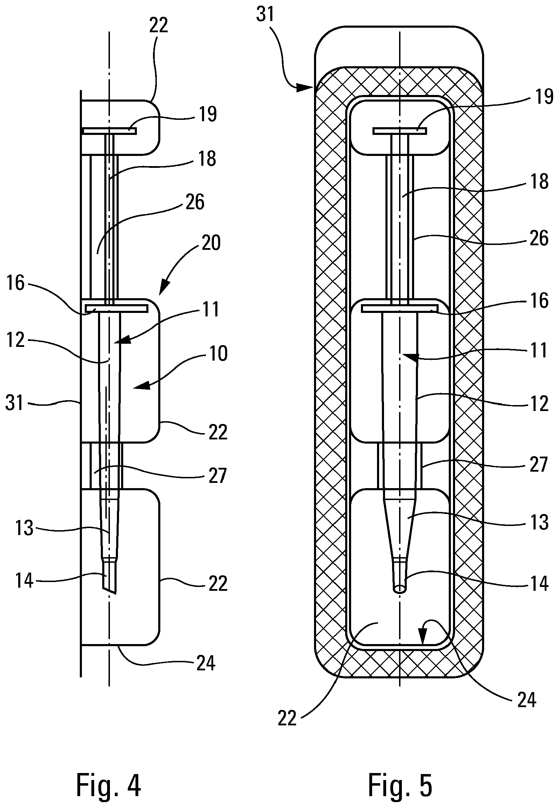

Les figures 3 à 5 représentent un second mode de réalisation. Dans ce second mode de réalisation, le dispositif de conditionnement 20 comporte un réservoir 22 réalisé sous la forme d'un blister fermé par une membrane étanche 31, notamment en aluminium. L'exemple de la figure 3 montre un réservoir du type blister de forme externe assez proche de celle du premier mode de réalisation représenté sur les figures 1 et 2, alors que les figures 4 et 5 montrent un mode de réalisation totalement différent, dans lequel la surface couverte par la membrane 31 est beaucoup plus grande. A nouveau, la forme externe du réservoir 22 proprement dite peut être différente de celle représentée. Avantageusement, comme pour le premier mode de réalisation, ce blister ou réservoir 22 est de préférence sensiblement rigide et avantageusement relativement transparent. Il comporte également avantageusement au moins un épaulement 26 adapté à éviter un contact entre l'embout d'éjection 14 de l'injecteur et les parois du réservoir 22.

Dans l'exemple des figures 4 et 5, cet épaulement 26 est formé par une partie rétrécie 26 du blister qui interconnecte deux parties agrandies 22 disposées au niveau d'une part de la bride radiale 16 du corps 11 de l'injecteur 10 et d'autre part au niveau de la surface d'appui 19 du piston d'actionnement 18 de l'injecteur. L'exemple des figures 4 et 5 montre également une troisième partie de dimension agrandie 22, située au niveau de l'embout d'éjection 14, afin de garantir l'absence de tout contact entre la paroi du réservoir et cet embout d'éjection 14. Un rétrécissement approprié 27 peut avantageusement être prévu entre les seconde et troisième parties agrandies 22 comme représentées sur les figures 4 et 5.

Dans son mode de réalisation préféré, dans lequel le réservoir est sensiblement rigide, la présente invention garantit une meilleure stérilité procurée par un système étanche rigide. En raison des écarts de pression et de température qui se produisent en fin d'autoclavage, la stérilisation est mieux supportée dans le cadre d'un réservoir rigide. Il est alors possible d'effectuer un vide de séchage pour garantir l'intégrité de l'enveloppe de protection externe, notamment en cas de double emballage stérile. L'absence de fuite de liquide par évaporation à travers la paroi du récipient ou réservoir est également avantageuse. Par comparaison, la paroi mince d'un récipient déformable, tel qu'une poche souple, est beaucoup plus perméable, ce qui peut être plus préjudiciable au maintien à long terme des propriétés de l'injecteur. De même, la plus grande solidité du réservoir de l'invention vis-à-vis des chocs et des effractions accidentelles, notamment lors du transport ou de manipulations, est avantageuse. Il en est de même de l'absence d'ambiguïtés quant à l'intégrité du réservoir contenant l'injecteur au moment de son utilisation. En effet, il n'y a pas de suspicion possible de microfuites, comme cela peut être le cas avec un blister légèrement froissé.

La présente invention est particulièrement adaptée à être utilisée avec un injecteur dans lequel la lentille est déjà prédisposée à l'intérieur du corps 11. Avantageusement, cette lentille est disposée dans le corps 11 de manière non-déformée et elle se déforme dans la partie tronconique 13 par la pression exercée par l'utilisateur sur le piston 18. Cette mise en oeuvre rend l'utilisation de l'injecteur très simple pour l'utilisateur finale, puisqu'il n'a qu'à ouvrir le dispositif de conditionnement 20, à saisir la surface d'actionnement 19 du piston 18 pour sortir l'injecteur du dispositif de conditionnement 20, puis à actionner cet injecteur pour éjecter la lentille qu'il contient. Il n'y a ici nul besoin de placer la lentille dans l'injecteur avant son utilisation, ni directement, ni par l'intermédiaire d'un dispositif accessoire.

D'autres modifications sont également possibles pour l'homme du métier sans sortir du cadre de la présente invention tel que défini par les revendications annexées.

## Revendications

1. Ensemble comportant un injecteur (10) de lentille intraoculaire comportant un corps (11) qui contient une lentille intraoculaire, et un dispositif de conditionnement (20), ledit dispositif de conditionnement (20) étant destiné à recevoir et stocker ledit injecteur (10) avant son utilisation, ledit dispositif de conditionnement (20) étant réalisé dans un matériau étanche à l'eau, et étant rempli d'une solution de stockage pour maintenir au moins le corps (11) dudit injecteur (10) et la lentille intraoculaire en immersion totale pendant toute la durée du stockage, ledit dispositif de conditionnement (20) comportant un réservoir sensiblement rigide (21, 22) et une ouverture (25) pour mettre en place et retirer ledit injecteur (10), ledit réservoir (21, 22) comportant au moins un épaulement interne (26, 27) coopérant avec une partie de l'injecteur (10) pour le centrer et maintenir son extrémité d'éjection (14) de la lentille en éloignement des parois dudit réservoir (21, 22), ledit dispositif de conditionnement (20) comportant un réservoir du type blister (22) fermé par une membrane étanche (31) notamment en aluminium

2. Ensemble selon la revendication 1, dans lequel ladite solution de stockage est de l'eau, une solution saline physiologique, ou une solution viscoélastique.

3. Ensemble selon l'une la revendication 1 ou 2, dans lequel ledit matériau est du verre.

4. Ensemble selon la revendication 1 ou 2, dans lequel ledit matériau est un matériau synthétique à haute étanchéité à la vapeur.

5. Ensemble selon l'une quelconque des revendications précédentes, dans lequel le remplissage du dispositif de conditionnement avec la solution de stockage est réalisé de telle sorte que la totalité du corps (11) de l'injecteur (10) est immergée dans toutes les positions dudit dispositif de conditionnement.

6. Ensemble selon l'une quelconque des revendications précédentes, dans lequel ledit injecteur (10) comporte un corps (11) comprenant une partie environ cylindrique (12) recevant la lentille de manière sensiblement non-déformée, une partie intermédiaire environ tronconique (13) pour plier la lentille, et une partie d'extrémité d'éjection de la lentille (14), l'injecteur (10) comprenant également des moyens de distribution (18) déplaçables manuellement dans ledit corps (11) pour déplacer la lentille de la partie environ cylindrique (12) du corps (11) vers la partie d'extrémité d'éjection (14) du corps (11), et pour ensuite l'éjecter, ledit corps (11) comportant à son extrémité opposée à l'extrémité d'éjection (14) une bride radiale (16) adaptée à coopérer avec un épaulement interne (26) prévu dans le dispositif de conditionnement (20) pour positionner ledit injecteur (10) dans ledit dispositif de conditionnement de telle sorte que l'extrémité d'éjection est éloignée des parois du dispositif de conditionnement (20) et que le corps (11) est totalement immergé dans ladite solution de stockage, en toutes positions dudit dispositif de conditionnement (20).

7. Ensemble selon l'une quelconque des revendications précédentes, dans lequel ledit dispositif de conditionnement (20) est sensiblement transparent.

8. Ensemble selon l'une quelconque des revendications précédentes, adapté à être soumis à une stérilisation par chaleur humide, notamment dans un autoclave à pression de vapeur d'eau.

## Patentansprüche

1. Einheit, die einen Injektor (10) für eine Intraokularlinse umfasst, der einen Körper (11) aufweist, der die Intraokularlinse enthält, und eine Konditionierungsvorrichtung (20), wobei diese Konditionierungsvorrichtung (20) dazu bestimmt ist, den Injektor (10) vor seinem Gebrauch aufzunehmen und zu lagern, wobei die Konditionierungsvorrichtung (20) aus einem wasserdichten Material hergestellt und mit einer Lagerungslösung gefüllt ist, um zumindest den Körper (11) des Injektors (10) und die Intraokularlinse während der Dauer der Lagerung vollständig eingetaucht aufzubewahren, wobei die Konditionierungsvorrichtung (20) einen im Wesentlichen starren Behälter (21, 22) und eine Öffnung (25) aufweist, die dazu dient, den Injektor (10) an seinen Platz zu bringen und zu entnehmen, wobei der Behälter (21, 22) wenigstens eine innenliegende Schulter (26, 27) umfasst, die mit einem Teil des Injektors (10) zusammenwirkt, um diesen zu zentrieren und sein zum Ausstoßen der Linse dienendes Ende (14) von den Wänden des Behälters (21, 22) im Abstand zu halten, wobei die Konditionierungsvorrichtung (20) einen Blisterbehälter (22) umfasst, der durch eine dichte Membran (31), die insbesondere aus Aluminium besteht, verschlossen ist.

2. Einheit nach Anspruch 1, bei der die Lagerungslösung Wasser, eine physiologische Salzlösung oder eine viskoelastische Lösung ist.

3. Einheit nach Anspruch 1 oder 2, **dadurch gekennzeichnet, dass** das Material Glas ist.

4. Einheit nach Anspruch 1 oder 2, **dadurch gekennzeichnet, dass** das Material ein synthetisches Material mit hoher Dampfdichtigkeit ist.

5. Einheit nach einem der vorhergehenden Ansprüche, bei welcher das Füllen der Konditionierungsvorrichtung mit der Lagerungslösung derart erfolgt, dass die Gesamtheit des Körpers (11) des Injektors (10) in allen Stellungen der Konditionierungsvorrichtung eingetaucht ist.

6. Einheit nach einem der vorhergehenden Ansprüche, bei welcher der Injektor (10) einen Körper (11) umfasst, der einen ungefähr zylindrischen Teil (12) aufweist, welcher die Linse so aufnimmt, dass sie im Wesentlichen nicht verformt ist, einen ungefähr stumpfkegeligen Zwischenteil (13) zum Verbiegen der Linse und einen zum Ausstoßen der Linse dienenden Endteil (14), wobei der Injektor (10) weiterhin Abgabeeinrichtungen (18) umfasst, die im Körper (11) von Hand verschiebbar sind, um die Linse aus dem ungefähr zylindrischen Teil (12) des Körpers (11) zu dem zum Ausstoßen der Linse dienenden Endteil (14) hin zu verschieben und die Linse danach auszustoßen, wobei der Körper (11) an seinem dem zum Ausstoßen dienenden Ende (14) gegenüberliegenden Ende einen radialen Flansch (16) umfasst, der geeignet ist, mit einer innenliegenden Schulter (26) zusammenzuwirken, die in der Konditionierungsvorrichtung (20) vorgesehen ist, um den Injektor (10) in der Konditionierungsvorrichtung derart zu positionieren, dass das zum Ausstoßen dienende Ende von den Wänden der Konditionierungsvorrichtung (20) beabstandet ist, und dass der Körper (11) in allen Positionen der Konditionierungsvorrichtung (20) vollständig in die Lagerungslösung eingetaucht ist.

7. Einheit nach einem der vorhergehenden Ansprüche, bei welcher die Konditionierungsvorrichtung (20) im Wesentlichen transparent ist.

8. Einheit nach einem der vorhergehenden Ansprüche, die geeignet ist, einem durch feuchte Hitze erfolgenden Sterilisierungsvorgang, insbesondere in einem Autoklaven unterworfen zu werden, der mit unter Druck stehendem Wasserdampf arbeitet.

## Claims

1. An assembly comprising a packaging device (20) and an intraocular lens injector (10) comprising a body (11) that contains an intraocular lens, said packaging device (20) being for receiving and storing said injector (10) prior to use, said packaging device (20) being made of a material that is waterproof, and being filled with a storage solution for maintaining at least the body (11) of said injector (10) and the intraocular lens in total immersion throughout the duration of storage, said packaging device (20) comprising a substantially rigid container (21, 22) and an opening (25) for enabling said injector (10) to be installed and removed, said container (21, 22) including at least one internal shoulder (26, 27) co-operating with a portion of the injector (10) to center and maintain its lens ejector end (14) away from the walls of said container (21, 22), said packaging device (20) comprising a blister-pack container (22) closed by a leakproof membrane (31), in particular an aluminum membrane.

2. An assembly according to claim 1, in which said storage solution is water, a physiological saline solution, or a viscoelastic solution.

3. An assembly according to claim 1 or claim 2, in which said material is glass.

4. An assembly according to claim 1 or claim 2, in which said material is a synthetic material presenting a high degree of leakproofing against water vapor.

5. An assembly according to any preceding claim, in which the packaging device is filled with the storage solution in such a manner that the body (11) of the injector (10) is fully immersed in all positions of said packaging device.

6. An assembly according to any preceding claim, in which said injector (10) comprises a body (11) having an approximately cylindrical portion (12) receiving the lens in substantially non-deformed manner, an approximately frustoconical intermediate portion (13) for folding the lens, and a lens-ejector end portion (14), the injector (10) also comprising dispenser means (18) manually movable within said body (11) to move the lens from the approximately cylindrical portion (12) of the body (11) towards the ejector end portion (14) of the body (11), and subsequently eject it, said body (11) having at its end remote from the ejector end (14) a radially-projecting flange (16) suitable for co-operating with an internal shoulder (26) provided in the packaging device (20) for positioning said injector (10) in said packaging device in such a manner that its ejector end is held apart from the walls of the packaging device (20) and the body (11) is completely immersed in said storage solution, in any position of said packaging device (20).

7. An assembly according to any preceding claim, in which said packaging device (20) is substantially transparent.

8. An assembly according to any preceding claim, adapted to be subjected to sterilization by wet heat, in particular in a steam pressure autoclave.
